# EUROPÄISCHE PATENTSCHRIFT

(11) **EP 0 946 599 B1**
(45) Veröffentlichungstag und Bekanntmachung des Hinweises auf die Patenterteilung: **28.02.2001**
(21) Anmeldenummer: 97954940.9
(22) Anmeldetag: 22.12.1997
(51) Int. Cl.: C08B 37/14

(54) **VERFAHREN ZUR VERBESSERUNG DER VERWERT- UND VERARBEITBARKEIT VON GUAR-ENDOSPERM UND DIE DANACH ERHALTENEN ERZEUGNISSE**
METHOD FOR IMPROVING THE EXPLOITABILITY AND PROCESSABILITY OF GUAR ENDOSPERM AND PRODUCTS OBTAINED USING SAID METHOD
PROCEDE POUR L'AMELIORATION DE L'EXPLOITABILITE ET DE L'OUVRABILITE D'ENDOSPERME DE GUAR, ET PRODUIT OBTENU SELON CE PROCEDE

(30) Priorität: 23.12.1996 DE 19654251
(43) Veröffentlichungstag der Anmeldung: 06.10.1999
(73) Patentinhaber: Rhodia Acetow GmbH, 79108 Freiburg (DE)
(72) Erfinder: Karstens, Ties, 79268 Bötzingen (DE); Stein, Armin, 79341 Kenzingen (DE)
(74) Vertreter: Hagemann, Heinrich, Dr.rer.nat., Dipl.-Chem.
(86) Internationale Anmeldenummer: EP9707230
(87) Internationale Veröffentlichungsnummer: WO9828337

(56) Entgegenhaltungen:
- EP-A- 0 048 612
- DE-A- 2 441 012
- DE-A- 3 331 701
- DE-A- 19 611 416
- DE-B- 1 261 384
- US-A- 2 496 670

## Beschreibung

Die Erfindung betrifft ein Verfahren zur Verbesserung der Verwert- und Verarbeitbarkeit von Guar-Endosperm und die danach erhältlichen Erzeugnisse in Form ammoniakexplodierter Guar-Endospermhälften (Guarsplits), Guarmehl und Guaranpulver.

Guarmehl wird in der Lebensmittelindustrie als Stabilisator für Speiseeis und einige Weichkäse, als Binde- und Verdickungsmittel für Soßen und ähnliche Erzeugnisse sowie auch in der kosmetischen Industrie verwendet. In der Technik wird Guarmehl zum Appretieren und Schlichten von Textilien und als Verdickungsmittel für Textildruckpasten verwendet. Eine große Menge Guarmehl wird auch in der Papierindustrie als Holländerzusatz zur Erzeugung stärkerer Papiere verwendet.

Der Hauptbestandteil von Guarmehl ist Guaran. Guaran ist ein Galactomannan, welches aus etwa 36% D-Galactose und 64% Mannose besteht. Die Mannoseeinheiten sind in Pyranoseform β-1,4-glycosidisch zu langen Hauptketten miteinander verknüpft, an denen die Galactoseeinheiten in Pyranoseform durch α-1,6-glycosidische Bindungen fixiert sind. Beim Guaran trägt jeder zweite Mannosebaustein der Hauptkette eine Galactoseseitengruppe. Das mittlere Molekulargewicht von Guaran ist meist bedeutend größer als 200.000.

Guaran ist im Endosperm des Samens der Guarbohne, Cyamopsis Tetragonoloba, enthalten, die in Indien verbreitet ist und seit 1944 in den USA in größerem Maße angebaut wird. Das Endosperm ist ein Nahrungsspeicher für die Entwicklung des Keimlings während der Keimung. Weil Guar eine zweikeimblättrige Pflanze ist, liegen in jedem Samen zwei Endospermhälften vor. Diese Endospermhälften umgeben den Keimling und sind ihrerseits von einer Samenschale umgeben, die üblicherweise von hellbrauner Farbe ist. Das Endosperm selbst besteht aus einer Zell-Schicht, dem Aleuron, und aus einer Nähr- und Speichersubstanz für den Keimling, dem Guaran. Die Zellen der Aleuronschicht enthalten viele Aleuronkörner, d.h. eingedickte Eiweißvakuolen. In den Aleuronzellen werden bei der Keimung des Guarsamens Enzyme synthetisiert und in das Endosperm abgegeben, um die Reservestoffe zu mobilisieren. Die beiden vorherrschenden Enzymaktivitäten sind α-Galactosidase- und β-Mannase-Aktivität.

Die Entfernung der Samenschale und des Keimlings geschieht technisch durch Mahlschritte und mechanische Aussortierung. Man nutzt hierbei die unterschiedliche Härte der Samenbestandteile. Die mehrstufigen Mahl- und Siebschritte werden oft mit anderen mechanischen Behandlungen zum Aufbrechen der Samen und Sortieren der Bestandteile kombiniert. Es existieren verschiedene Arten von Mühlen, die in Verbindung mit Röstvorgängen oder der Behandlung des Samens mit Wasser oder Säure eingesetzt werden können. Auf eine gründliche Entfernung des Keimlings muß bei Verwendung für Lebensmittel besonders geachtet werden. Das gereinigte Endosperm wird unter der Bezeichnung "Guarsplits" vertrieben.

Die Guarsplits werden üblicherweise zu einem Pulver gemahlen, das Guarmehl oder Guar Gum Powder genannt wird. Die proteinhaltige Endospermhülle der Guarsplits wird hierbei nicht entfernt. Bei bestimmten Anwendungen stört der durch die proteinhaltige Hülle eingebrachte Proteinanteil im Guarmehl. Es existiert daher ein Bedürfnis nach einem einfachen und effizienten Verfahren, nach dem Guaran in sehr reiner Form aus den Guarsplits isoliert werden kann.

Die Mahlung der Guarsplits ist außerdem mit einem erheblichen elektrischen Energieaufwand verbunden. Die Mahlbedingungen beeinflussen ferner die Viskosität der wäßrigen Lösung von Guaran bzw. dessen Derivaten. Es besteht daher ein Bedürfnis nach einem Verfahren, bei dem keine Mahlung der Guarsplits erforderlich ist.

Wäßrige Lösungen von handelsüblichem Guarmehl sind üblicherweise trüb. Die Trübung wird hauptsächlich durch das Vorliegen unlöslicher Anteile des Endosperms hervorgerufen. Aus Guarmehl hergestellte Derivate zeigen zwar im allgemeinen eine verbesserte Löslichkeit und Klarheit der Lösung. Die verbesserte Klarheit rührt von der Derivatisierung und Solubilisierung unlöslicher Samenverunreinigung her. Für bestimmte Anwendungen sind jedoch auch die Eigenschaften von derivatisiertem Guarmehl unzureichend. So zeigt carboxymethylierter Guar eine relativ hohe Strukturviskosität mit einem nur schwach ausgeprägten Newtonschen Bereich bei niedrigen Scherraten. Bei Verwendung von carboxymethlyiertem Guar als Verdickungsmittel beim Textildruck stellt sich bei den handelsüblichen Produkten eine schlechte Auswaschbarkeit ein. Die Ursache liegt vermutlich an einer inhomogenen Substituentenverteilung, die ihrerseits davon herrührt, daß die Derivatisierungen in heterogener Reaktion an den gemahlenen Guarsplits durchgeführt werden. Selbst durch Mahlung auf eine sehr kleine Teilchengröße kann dieser Nachteil nicht vollständig behoben werden. Es besteht daher ein Bedürfnis nach einem Guarerzeugnis, das vollständig wasserlöslich ist bzw. in homogener Reaktion derivatisiert werden kann.

Der Erfindung liegt daher die Aufgabe zugrunde, Vorschläge zu unterbreiten, mit denen die oben angesprochenen Bedürfnisse befriedigt werden können. Insbesondere soll die Verwert- und Verarbeitbarkeit von Guar-Endospermhälften (Guarsplits) verbessert werden, so auch die Vermahlbarkeit zu Guarmehl. Darüber hinaus soll reines Guaran aus Guarsplits einfach und effizient isoliert werden können, wobei kein Mahlen der Guarsplits erforderlich ist und wobei das Guaran vollständig wasserlöslich ist und in homogener Reaktion derivatisiert werden kann.

Erfindungsgemäß wird diese Aufgabe gelöst durch ein Verfahren zur Verbesserung der Verwert- und Verarbeitbarkeit von Guar-Endosperm, bei dem Guar-Endospermhälften (Guarsplits) mit flüssigem Ammoniak bei einem gegenüber Atmosphärendruck erhöhten Ausgangsdruck und bei einer Temperatur von mindestens etwa 25°C in Kontakt gebracht werden, wobei die Menge des flüssigen Ammoniaks zumindest zur Benetzung der Oberfläche der Guar-Endospermhälften ausreicht, und das dem System Guar-Endospermhälften/flüssiges Ammoniak zur Verfügung stehende Volumen unter Senken des Drucks um mindestens etwa 0,5 MPa (5 bar) explosisonsartig vergrößert wird und hierdurch die Hülle der Guar-Endospermhälften aufgerissen wird.

Die WO 96/30411 offenbart ein Verfahren zur Aktivierung von Polysacchariden durch Ammoniakexplosion. In einem Beispiel wird Guarmehl mit flüssigem Ammoniak behandelt und explodiert. Die WO 96/30411 legt nicht nahe, anstelle von Guarmehl intakte Guar-Endospermhälften als Ausgangsmaterial einzusetzen.

Bei dem Guar-Endospermausgangsmaterial handelt es sich vorzugsweise um Endospermhälften, die nicht wesentlich vorzerkleinert sind, d.h. im wesentlichen intakte Guarsplits.

Bei der Behandlung der Guarsplits mit flüssigem Ammoniak kann der flüssige Ammoniak die die Guarsplits umgebende Hülle penetrieren und in den Polysaccharidkern eindringen. Bei der anschließenden Explosion nimmt das Volumen des eingedrungenen Ammoniaks schlagartig stark zu. Der gasförmige Ammoniak kann nicht mehr schnell genug durch die Hülle entweichen und führt zu einem Aufreißen der Oberfläche der Guarsplits. Das in den nativen Guarsplits enthaltene Guaran ist mikrokristallin und weist im allgemeinen einen Kristallinitätsgrad von ca. 20 bis 30% auf. Unter Einwirkung des flüssigen Ammoniaks erfolgt eine zumindest partielle Quellung der Polysaccharidsubstanz. Intermolekulare Wasserstoffbrückenbindungen zwischen den Molekülketten werden gelöst, da das Ammoniakmolekül mit den Hydroxylgruppen der Nachbarmoleküle konkurriert. Durch die Explosion erfolgt eine Verdampfung des zwischen den Molekülketten befindlichen Ammoniaks. Die Molekülketten, deren intermolekulare Wasserstoffbrückenbindungen zuvor gelöst worden sind, werden auseinandergerissen. Dies führt zu einem Aufschluß von normalerweise für Reagentien nur schlecht zugänglichen Bereichen. Insbesondere wird der Polysaccharidanteil durch die Ammoniakexplosion wasserlöslich. Das Guaran in den explodierten Guarsplits ist nicht mehr kristallin, sondern amorph.

Wenn im Zusammenhang mit dem erfindungsgemäßen Verfahren von "explosionsartig" gesprochen wird, dann ist dieser Begriff eng zu sehen. Vorzugsweise erfolgt die explosionsartige Volumenvergrößerung innerhalb einer Zeit von weniger als 1 s, insbesondere weniger als 0,5 s. Die Ammoniakexplosion des erfindungsgemäßen Verfahrens kann dabei chargenweise oder kontinuierlich geschehen. Bei kontinuierlicher Verfahrensführung wird bei der Betrachtung auf eine inkrementelle Menge Guar-Endospermhälften/flüssiges Ammoniak abgestellt. Die Guar-Endospermhälften und das flüssige Ammoniak werden vorzugsweise in einer Druckeinrichtung in Kontakt gebracht und das System Guar-Endospermhälften/flüssiges Ammoniak durch Überführen in einen Explosionsraum mit gegenüber der Druckeinrichtung größerem Volumen entspannt. Vorzugsweise liegt der Ausgangsdruck zwischen etwa 0,5 und 4,6 MPa (5 und 46 bar) und insbesondere zwischen etwa 2,5 und 3,0 MPa (25 und 30 bar). Der Mindestdruckabfall von 0,5 MPa (5 bar) ist kritisch. Wird er unterschritten, dann wird das Erfindungsziel nicht erreicht. Der obere Grenzwert von etwa 4,6 MPa (46 bar) führt bei seiner Überschreitung nicht zu weitergehenden Vorteilen. Seine Einstellung bedingt einen relativ großen apparativen Aufwand, so daß eine weitere Anhebung unter praktischen Erwägungen nicht sinnvoll ist. Mit dem angegebenen Druckrahmen korreliert die Temperatur von etwa 25 bis 85°C bzw. 55 bis 65°C. Vorzugsweise wird der Ausgangsdruck in dem System Guar-Endospermhälften/flüssiges Ammoniak explosionsartig um mindestens etwa 1 MPa (10 bar) und insbesondere etwa 3,0 MPa (30 bar) gesenkt. Vorzugsweise erfolgt die Explosion in einen Explosionsraum, der unter Vakuum gehalten wird.

In die Druckeinrichtung muß eine ausreichende Menge Ammoniak eingepreßt werden, so daß unter den erfindungsgemäß erforderlichen Druck- bzw. Temperaturbedingungen flüssiges Ammoniak vorhanden ist und zumindest die Oberfläche der Guar-Endospermhälften benetzt ist. Vorzugsweise entfallen auf 1 Masse-Teil Guar-Endospermhälften mindestens etwa 1 Masse-Teil flüssiges Ammoniak, insbesondere mindestens etwa 2 Masse-Teile und ganz besonders bevorzugt etwa 5 bis 10 Masse-Teile flüssiges Ammoniak.

Der Schritt der Ammoniakexplosion des erfindungsgemäßen Verfahrens kann diskontinuierlich oder kontinuierlich durchgeführt werden. Bei diskontinuierlicher Verfahrensführung weist die Apparatur im wesentlichen einen Druckbehälter, der mit dem zu behandelnden Gut befüllt werden kann, und einen über ein Ventil daran angeschlossenen Auffang- bzw. Expansionsbehälter auf. Es gilt hierbei zu beachten, daß das Ventil im geöffneten Zustand eine große lichte Öffnung aufweist, damit sich beim Explosionsvorgang die Guar-Endospermhälften nicht stauen und nicht lediglich Ammoniak entweicht. Der Expansionsbehälter weist gegenüber dem Druckbehälter ein vielfaches Volumen auf, beispielsweise beträgt das Volumen des Druckbehälters 1 l und das Volumen des Expansionsbehälters 30 l. Der Druckbehälter ist mit einer Zuführleitung für Ammoniak, gegebenenfalls unter Zwischenschaltung einer druckerhöhenden Vorrichtung, verbunden. Zur weiteren Druckerhöhung kann außerdem eine Zuführleitung für Inertgase, z.B. Stickstoff, vorgesehen sein.

In kontinuierlicher Weise könnte das Verfahren unter Heranziehen eines rohr- oder zylinderförmigen, druckfesten Reaktors durchgeführt werden, bei dem das Inkontaktbringen von Guar-Endospermhälften und flüssigem Ammoniak im Zylinder des Reaktors stattfindet und das imprägnierte Material mit Hilfe einer Förderschnecke als Pfropfen durch den Reaktor transportiert wird und intermittierend durch ein Ventil oder ein geeignetes System von Druckschleusen in einen Auffangraum ausgetragen wird.

Die Kontaktzeit zwischen dem flüssigen Ammoniak und den Guar-Endospermhälften ist nicht kritisch. Sie beträgt vorzugsweise mindestens etwa 1 min, in der Regel 4 bis 8 min oder mehr. Nach der Explosion enthält das anfallende Material im allgemeinen weniger als etwa 2 Gew.-% Ammoniak. Der Restammoniakgehalt ist für das weitere Verfahren unkritisch.

Eine erste vorteilhafte Weiterbildung des erfindungsgemäßen Verfahrens besteht darin, daß das (ammoniak-) explodierte Material mit einem Extraktionsmittel behandelt wird, so daß das Guaran im wesentlichen in die Lösung geht und die Endospermhüllen im wesentlichen ungelöst bleiben, die Endospermhüllen abgetrennt werden und, fakultativ, aus der Guaranlösung Guaran gewonnen wird.

Bevorzugte Extraktionsmittel für die Behandlung des ammoniakexplodierten Materials sind wäßrige Medien, insbesondere Wasser, oder andere Lösungsmittel mit vergleichbaren Lösungseigenschaften. Bei der Extraktion des ammoniakexplodierten Materials mit z.B. Wasser löst sich der Polysaccharidanteil der Guarsplits bereitwillig, während die die Splits umgebende Hülle unlöslich bleibt und nach üblichen Techniken, z.B. durch Filtration oder Zentrifugation, abgetrennt werden kann. Das explodierte Material wird vorzugsweise bei einer Temperatur von etwa 25 bis 95°C mit dem Extraktionsmittel behandelt.

Die wäßrige Guaranlösung kann als solche, z.B. zu Derivatisierungen in homogener wäßriger Phase, verwendet oder nach üblichen Verfahren getrocknet werden. Zur Trocknung sind insbesondere die Sprühtrocknung oder Trommeltrocknung geeignet. Das anfallende Pulver ist in Wasser unter Bildung sehr klarer Lösungen hervorragend löslich.

Nach dem erfindungsgemäßen Verfahren fällt also Guaran als wäßrige Lösung bzw. Pulver hervorragender Wasserlöslichkeit an, so daß zur Herbeiführung der Wasserlöslichkeit keine weitergehende Derivatisierung mehr erforderlich ist. Sofern im Einzelfall gewünscht, führt die Derivatisierung von erfindungsgemäß hergestelltem Guaran zu Produkten mit überraschend verbesserten Eigenschaften, da durch die verbesserte Akzessibilität für die Derivatisierungsreagentien homogenere Derivatisierungsprodukte erhalten werden. Deren Herstellung ist mit einem geringeren Chemikalieneinsatz und einem geringeren Anfall von Nebenprodukten möglich. Die Homogenität der Substituentenverteilung ist dabei höher. Durch das erfindungsgemäße Verfahren erleidet das Guaran keinen nennenswerten DP-Abbau. Durch Röntgenbeugungsspektren konnte nachgewiesen werden, daß das ursprünglich zumindest teilkristalline Guaran nunmehr amorph ist. Das Molekulargewicht liegt merklich unter dem des nativen Ausgangsmaterials. Als Rahmen für das erfindungsgemäß durch Extraktion aus den ammoniakexplodierten Guarsplits gewonnene Guaran ist ein Molekulargewicht von etwa 1,5 bis 2,5 Millionen, insbesondere etwa 1,8 bis 2,2 Millionen zu setzen. Der Anteil der erfindungsgemäß ammoniakexplodierten Guarsplits an wasserlöslicher Fraktion liegt zwischen etwa 53 und 59 Gew.-%, insbesondere zwischen etwa 56 und 66 Gew.-%. Werden die erfindungsgemäß erhaltenen ammoniakexplodierten Guarsplits einem üblichen Mahlen auf eine Teilchengröße von etwas mehr als etwa 100 µm unterzogen, dann führt dies zu einer weiteren Senkung des Molekulargewichts des Guarans in dem Mahlgut. Hierbei ergeben sich Molekulargewichtswerte von etwa 1,4 bis 1,65 Millionen, während der wasserlösliche Anteil etwa 65 bis 77 Gew.-% beträgt.

Werden schließlich die Guarsplits getrocknet und dann bis auf eine Teilchengröße von etwa 100 µm gemahlen, dann tritt eine besonders starke Senkung des Molekulargewichts des Guarans ein. In diesen Fällen liegt dessen Molekulargewicht zwischen etwa 450 000 und 900 000, wobei der wasserlösliche Anteil zwischen etwa 71 und 85 Gew.-% liegt.

Die erfindungsgemäß erhaltenen bzw. ammoniakexplodierten Guarsplits sind darüber hinaus wie folgt zu kennzeichnen: Sie zeigen eine durch die Ammoniakexplosion aufgerissene Hülle. Diese Hülle bleibt nach der Ammoniakexplosion im wesentlichen chemisch unverändert. Das in Guarsplits enthaltene bzw. daraus gewonnene Guaran zeigt gesteigerte Reaktivität bei chemischen Umsetzungen, so bei der Veretherung (Carboxymethylierung), insbesondere bei der Silylierung. Das noch eingeschlossene Guaran ist porös und amorph. Die Porösität kann man wie folgt beschreiben: Die Ammoniakexplosion der Guarsplits schafft Vakuolen (Hohlräume) im Inneren der Splits, die durch den gasförmig entweichenden Ammoniak mit der Oberfläche über Kanäle in Verbindung stehen. In gequollenem Zustand sind die Splits bis zum Dreifachen ihres Volumens gequollen. Die Porösität läßt sich durch rasterelektronische Aufnahmen nachweisen, wozu auf die anliegenden rasterelektronischen Darstellungen verwiesen sei. Das Molekulargewicht liegt in dem oben bezeichneten Rahmen. Der wasserlösliche Anteil, der im wesentlichen auf Guaran zurückgeht, wurde ebenfalls oben rahmenmäßig bezeichnet. Darüber hinaus hat es sich gezeigt, daß die Quellbarkeit der ammoniakexplodierten Guarsplits gegenüber den nicht-explodierten nativen Materialien stark zunimmt, was auch für die unterschiedlichsten Medien gilt, so z.B. Wasser oder ein Gemisch "Wasser + Soda" bei Raumtemperatur oder auch bei erhöhten Temperaturen. Bei Messungen bei einer Temperatur von 23°C in einem wäßrigen Medium hat es sich gezeigt, daß erfindungsgemäß erhaltene Guarsplits eine um nahezu 100 % größere Quellung als die nativen Vergleichsprodukte nach einer Quellzeit von 60 Minuten zeigte. Bei einem vorgebenen Quellvolumen bedeutet das, daß dieses von erfindungsgemäß erhaltenen Guarsplits in der halben Zeit erreicht wird. Wenn in Einzelfällen im Hinblick auf bestimmte Verwendungen des Guarans das Trocknen zur Entfernung von Restammoniak nicht ausreicht, dann läßt sich dieses Restammoniak dadurch weitergehend und ausreichend entfernen, indem ein Austausch mit z.B. Isopropanol durchgeführt wird. Ferner hat es sich gezeigt, daß die ammoniakexplodierten Guarsplits weniger flüchtigen Anteil als die Vergleichssplits aufweisen. Darüber hinaus ist die Klarheit der Lösung ammoniakexplodierter Guarsplits geringfügig höher als die der Vergleichssplits. Diese verbesserte Transparenz läßt vermuten, daß die ammoniakexplodierten Guarsplits weniger wasserunlösliche Materialien enthalten.

Eine weitere Fortbildung des erfindungsgemäßen Verfahrens besteht darin, die durch die erfindungsgemäß genutzte Ammoniakexplosion erhaltenen explodierten Guarsplits in üblicher Weise zu Guarmehl zu vermahlen. Dabei ist es im allgemeinen bevorzugt, daß während des Mahlvorgangs Wasser zugesetzt ist. Sollte im Einzelfall kein Wasser beim Mahlen zugegen sein, dann wird dem Vermahlen zweckmäßigerweise ein Trocknen vorgeschaltet.

Die Erfindung soll nun durch die nachfolgenden Beispiele näher erläutert werden.

### Beispiel 1

300 g handelsübliche Guarsplits wurden in einen Autoklaven eines Volumens von 1 1 mit einer Doppelwand zur Damptbeheizung gegeben. Anschließend wurden 500 g flüssiges Ammoniak über ein Ventil in den Autoklaven gepreßt. Durch die zusätzliche Dampfheizung des Autoklavs wurde die Temperatur auf 66°C angehoben. Hierbei stellte sich innerhalb des Autoklaven ein Druck von ca. 2 MPa (20 bar). Das System wurde 60 s unter diesen Bedingungen gehalten. Anschließend wurde es durch Öffnung eines Ventils (Durchmesser der Öffnung : 4 cm) schlagartig und gänzlich in einen Auffangbehälter eines Volumens von 30 l entspannt. Der Ammoniakgehalt des im Auffangbehälter anfallenden Erzeugnisses lag bei etwa 0,8 Gew.-%, bezogen auf das Guar.

Man erkennt in den Figuren 1 bzw. 2, daß die Ammoniakexplosion zu einem Aufreißen der Oberfläche der Splits führt, während beim unbehandelten Split die Oberfläche glatt und geschlossen erscheint.

### Beispiel 2

8 g der ammoniakexplodierten Guarsplits wurden in ein temperiertes Doppelmantelgefäß gebracht, das 192 g Wasser von 50°C enthielt. Das Gefäß war mit einem Rührer (Heidolph RZR2101 electronic Antrieb) ausgerüstet, der es ermöglichte, das Drehmoment der gerührten Masse zu verfolgen. Der verwendete Rührer war ein Flächenrührer, es wurde bei 250 U/min gearbeitet. Der Verlauf des Drehmoments entspricht dem Widerstand, den die wäßrige Lösung auf das Rührwerkzeug ausübt und damit der Viskosität der wäßrigen Lösung. Die Viskosität hängt von der gelösten Menge an Guaran ab und steigt mit zunehmender Konzentration dieses Biopolymeren. Nach 2,5 h erreicht der Verlauf des Drehmomentes ein Plateau. Hieraus kann geschlossen werden, daß der Lösevorgang beendet ist. Bei visueller Beobachtung sind ungelöste Partikel (Endospermhüllen) erkennbar, die nach dem Abstellen des Rührers auf den Boden des Gerätes absinken. Die überstehende Lösung ist klar und kann abdekantiert werden.

In Vergleichsversuchen wurden unbehandelte Guarsplits (Handelsform) unter gleichen Bedingungen in Wasser gerührt und der Verlauf des Drehmoments verfolgt. Ferner wurden unbehandelte Splits in 4%igem Ammoniakwasser bei 25°C gerührt. Der jeweilige Verflauf des Drehmoments ist in Figur 3 dargestellt. Bei den Vergleichsversuchen kam es zu keinem nennenswerten Viskositätsanstieg. Dies deutet darauf hin, daß keine Auflösung von Guaran aus den unbehandelten Guarsplits erfolgte.

Weitergehende Bestimmungen ergaben folgendes: Durch Gelchromatographie-Analyse wurde festgestellt, daß die ammoniakexplodierten Guarsplits Guaran eines Molekulargewichts von 1 996 000 und einen wasserlöslichen Anteil von 61 Gew.-% enthielten. Die entsprechenden Werte bei den Vergleichssplits ergaben ein Molekulargewicht von 278 900 und 51 Gew.-%.

### Beispiel 3

Es sollte hier der Einfluß des Mahlens und gegebenenfalls des vorausgeschalteten Trocknens auf das Molekulargewicht des Guarans in dem erhaltenen Guaranmehl sowie der Anteil an wasserlöslicher Fraktion ermittelt werden. Herangezogen wurden daher native Guarsplits sowie ammoniakexplodierte Guarsplits nach Beispiel 2. In einem Fall wurde lediglich auf einen durchschnittlichen Teilchendurchmesser von mehr als 100 µm gemahlen. Dies erfolgte in einer sogenannten Kryomühle unter schonenden Bedingungen sowie unter Zusatz flüssigen Stickstoffs. Darüber hinaus wurde ein Versuch durchgeführt, bei dem das Mahlen auf einen durchschnittlichen Teilchendurchmesser von etwa 100 µm erfolgte, wobei bei einer Temperatur von 40°C über Nacht in einem Vakuumtrockenschrank getrocknet wurde. Danach ergaben sich die aus der nachfolgenden Tabelle ersichtlichen Daten:

**Tabelle**

| | Native Guarsplits | | Ammoniakbehandelte Guarsplits | |
|---|---|---|---|---|
| | Molekulargewicht | wasserlösliche Fraktion | Molekulargewicht | wasserlösliche Fraktion |
| Guarsplits | 2 789 000 | 51% | 1 996 000 | 61% |
| Guarsplits nach Mahlen ¹⁾ | 1 720 000 | 60% | 1 510 000 | 71% |
| Guarsplits nach Trocknen und Mahlen ²⁾ | 1 129 000 | 66% | 577 400 | 78% |

| | | | | |
|---|---|---|---|---|
| Anmerkungen: ¹⁾ Es erfolgt eine schonende Kryomahlung unter Zusatz von flüssigem Stickstoff auf einen Teilchendurchmesser > 100 µm. | | | | |
| ²⁾ Es erfolgt hier zunächst ein Trocknen bei 40°C über Nacht im Vakuum. Es schließt sich einen schonende Kryomahlung unter Zusatz von flüssigem Stickstoff auf eine durchschnitteliche Teilchengröße von 100 µm an. | | | | |

## Patentansprüche

1. Verfahren zur Verbesserung der Verwert- und Verarbeitbarkeit von Guar-Endosperm, bei dem Guar-Endospermhälften (Guarsplits) mit flüssigem Ammoniak bei einem gegenüber Atmosphärendruck erhöhten Ausgangsdruck und bei einer Temperatur von mindestens etwa 25°C in Kontakt gebracht werden, wobei die Menge des flüssigen Ammoniaks zumindest zur Benetzung der Oberfläche der Guar-Endospermhälften ausreicht, und das dem System Guar-Endospermhälften/flüssises Ammoniak zur Verfügung stehende Volumen unter Senken des Drucks um mindestens etwa 0,5 MPa (5 bar) explosisonsartig vergrößert wird und hierdurch die Hülle der Guar-Endospermhälften aufgerissen wird.

2. Verfahren nach Anspruch 1, dadurch gekennzeichnet, daß die explosionsartige Volumenvergrößerung innerhalb einer Zeit von weniger als 1 s durchgeführt wird.

3. Verfahren nach Anspruch 1 oder 2, dadurch gekennzeichnet, daß die Guar-Endospermhälften und das flüssige Ammoniak in einer Druckeinrichtung in Kontakt gebracht werden und das System Guar-Endospermhälften/flüssiges Ammoniak durch Überführen in einen Explosionsraum mit gegenüber der Druckeinrichtung größerem Volumen entspannt wird.

4. Verfahren nach mindestens einem der Ansprüche 1 bis 3, dadurch gekennzeichnet, daß der Ausgangsdruck zwischen etwa 0,5 und 4,6 MPa (5 und 46 bar) eingestellt wird.

5. Verfahren nach Anspruch 3 oder 4, dadurch gekennzeichnet, daß die Temperatur in der Druckeinrichtung vor der explosionsartigen Volumenvergrößerung zwischen etwa 25 und 85°C eingestellt wird.

6. Verfahren nach mindestens einem der vorhergehenden Ansprüche, dadurch gekennzeichnet, daß der Ausgangsdruck explosionsartig um mindestens etwa 1 MPa (10 bar) gesenkt wird.

7. Verfahren nach mindestens einem der vorhergehenden Ansprüche, dadurch gekennzeichnet, daß auf 1 Masse-Teil Guar-Endospermhälften mindestens 1 Masse-Teil flüssiges Ammoniak eingesetzt wird.

8. Verfahren nach mindestens einem der vohergehenden Ansprüche, dadurch gekennzeichnet, daß das explodierte Material mit einem Extraktionsmittel behandelt wird, so daß das Guaran im wesentlichen in die Lösung geht und die Endospermhülsen im wesentlichen ungelöst bleiben, die Endospermhüllen abgetrennt und, fakultativ, aus der Guaranlösung Guaran gewonnen wird.

9. Verfahren nach Anspruch 8, dadurch gekennzeichnet, daß ein wäßriges Extraktionsmittel, insbesondere Wasser, verwendet wird.

10. Verfahren nach Anspruch 8 oder 9, dadurch gekennzeichnet, daß das explodierte Material bei einer Temperatur von etwa 25 bis 95°C mit dem Extraktionsmittel behandelt wird.

11. Verfahren nach einem der Ansprüche 8 bis 10, dadurch gekennzeichnet, daß die Endospermhüllen durch Filtration oder Zentrifugation abgetrennt werden.

12. Verfahren nach einem der Ansprüche 8 bis 11, dadurch gekenntzeichnet, daß das Guaran durch Sprühtrocknen gewonnen wird.

13. Guaranpulver, erhältlich durch das Verfahren nach einem der Ansprüche 8 bis 12.

14. Verfahren nach Anspruch 1, dadurch gekennzeichnet, daß das ammoniakexplodierte Material zu Guarmehl vermahlen wird.

15. Ammoniakexplodierte Guar-Endospermhälfte (Guarsplits), erhältlich durch das Verfahren nach mindestens einem der Ansprüche 1 bis 7.

16. Guarmehl, erhältlich durch das Verfahren nach Anspruch 14.

## Claims

1. Process for improving the utilizability and processability of guar endosperm, in which guar endosperm halves (guar splits) are contacted with liquid ammonia at a starting pressure which is higher than atmospheric pressure and at a temperature of at least 25°C, the amount of liquid ammonia being at least sufficient to wet the surface of the guar endosperm halves, and the volume available to the system guar-endosperm halves/liquid ammonia being explosively increased with reduction of the pressure by at least about 0.5 MPa (5 bar) and as a result the hull of the guar endosperm halves being broken open.

2. Process according to Claim 1, characterized in that the explosive volume increase is carried out within a time of less than 1 s.

3. Process according to Claim 1 or 2, characterized in that the guar endosperm halves and the liquid ammonia are contacted in a pressure device and the system guar-endosperm halves/liquid ammonia is expanded by transfer into an explosion chamber having a volume greater than the pressure device.

4. Process according to at least one of Claims 1 to 3, characterized in that the starting pressure is set between about 0.5 and 4.6 MPa (5 and 46 bar).

5. Process according to Claim 3 or 4, characterized in that the temperature in the pressure device before the explosive volume increase is set at between about 25 and 85°C.

6. Process according to at least one of the preceding claims, characterized in that the starting pressure is decreased explosively by at least about 1 MPa (10 bar).

7. Process according to at least one of the preceding claims, characterized in that at least one part by mass of liquid ammonia is used per part by mass of guar endosperm halves.

8. Process according to at least one of the preceding claims, characterized in that the exploded material is treated with an extraction medium, so that the guarane essentially passes into the solution and the endosperm hulls remain essentially undissolved, the endosperm hulls are separated off and, optionally, guarane is produced from the guarane solution.

9. Process according to Claim 8, characterized in that an aqueous extraction medium, in particular water, is used.

10. Process according to Claim 8 or 9, characterized in that the exploded material is treated with the extraction medium at a temperature of about 25 to 95°C.

11. Process according to one of Claims 8 to 10, characterized in that the endosperm hulls are separated off by filtration or centrifugation.

12. Process according to one of Claims 8 to 11, characterized in that the guarane is produced by spray-drying.

13. Guarane powder, obtainable by the process according to one of Claims 8 to 12.

14. Process according to Claim 1, characterized in that the ammonia-exploded material is ground to form guar flour.

15. Ammonia-exploded guar endosperm halves (guar splits), obtainable by the process according to at least one of Claims 1 to 7.

16. Guar flour, obtainable by the process according to Claim 14.

## Revendications

1. Procédé pour accroître la capacité de mise en valeur et de mise en oeuvre d'endosperme de guar, dans lequel des moitiés d'endosperme de guar (Guarsplits) sont mises en contact avec de l'ammoniac liquide à une pression de départ élevée par rapport à la pression atmosphérique et à une température d'au moins environ 25°C, de manière que la quantité d'ammoniac liquide soit au moins suffisante pour mouiller la surface des moitiés d'endosperme de guar, et le volume dont le système moitiés d'endosperme de guar/ammoniac liquide dispose est agrandi par explosion avec abaissement de la pression d'au moins environ 0,5 MPa (5 bars), ce qui a pour effet de déchirer l'enveloppe des moitiés d'endosperme de guar.

2. Procédé suivant la revendication 1, caractérisé en ce que l'augmentation de volume par explosion s'accomplit en moins d'une seconde.

3. Procédé suivant la revendication 1 ou 2, caractérisé en ce que les moitiés d'endosperme de guar et l'ammoniac liquide sont mis en contact dans un dispositif sous pression et le système moitiés d'endosperme de guar/ammoniac liquide est détendu par transfert dans une chambre d'explosion de volume supérieur à celui du dispositif sous pression.

4. Procédé suivant au moins l'une des revendications 1 à 3, caractérisé en ce que la pression initiale est ajustée entre environ 0,5 et 4,6 MPa (5 et 46 bars).

5. Procédé suivant la revendication 3 ou 4, caractérisé en ce que la température dans le dispositif sous pression avant l'augmentation de volume par explosion est ajustée entre environ 25 et 85°C.

6. Procédé suivant au moins l'une des revendications précédentes, caractérisé en ce que la pression initiale est abaissée d'au moins environ 1 MPa (10 bars) par explosion.

7. Procédé suivant au moins l'une des revendications précédentes, caractérisé en ce qu'on utilise au moins une partie en masse d'ammoniac liquide par partie en masse de moitiés d'endosperme de guar.

8. Procédé suivant au moins l'une des revendications précédentes, caractérisé en ce que la matière explosée est traitée avec un agent d'extraction de manière que le guarane passe essentiellement en solution et que les enveloppes de l'endosperme restent essentiellement non dissoutes, les enveloppes de l'endosperme sont séparées et, facultativement, le guarane est obtenu à partir de sa solution.

9. Procédé suivant la revendication 8, caractérisé en ce qu'on utilise un agent aqueux d'extraction, en particulier l'eau.

10. Procédé suivant la revendication 8 ou 9, caractérisé en ce que la matière explosée est traitée avec l'agent d'extraction à une température d'environ 25 à 95°C.

11. Procédé suivant l'une des revendications 8 à 10, caractérisé en ce que les enveloppes de l'endosperme sont séparées par filtration ou centrifugation.

12. Procédé suivant l'une des revendications 8 à 11, caractérisé en ce que le guarane est obtenu par séchage par atomisation.

13. Guarane en poudre obtenu par le procédé suivant l'une des revendications 8 à 12.

14. Procédé suivant la revendication 1, caractérisé en ce que la matière explosée à l'ammoniac est broyée en farine de guar.

15. Moitiés d'endosperme de guar (Guarsplits) explosées à l'ammoniac, obtenues par le procédé suivant au moins l'une des revendications 1 à 7.

16. Farine de guar obtenue par le procédé suivant la revendication 14.
